# EUROPEAN PATENT APPLICATION

(11) **EP 3 189 818 A1**
(43) Date of publication of application: **12.07.2017**
(21) Application number: 15720187.2
(22) Date of filing: 20.01.2015
(51) Int. Cl.: A61F 5/442

(54) **DEFECATION CLEANER CUP COVER STRUCTURE AND DEFECATION CLEANER**

(30) Priority: 01.09.2014 CN 201410440233
(71) Applicant: BOE Technology Group Co., Ltd., Beijing 100015 (CN)
(72) Inventor: WEI, Qiang, Beijing 100176 (CN)
(74) Representative: Michalski Hüttermann & Partner Patentanwälte mbB
(86) International application number: PCT/CN2015/071102
(87) International publication number: WO 2016/033933

(57) **Abstract**

The invention discloses relates to the filed of sanitary ware, and in particular to a defecating device cup structure and a defecating device. The defecating device cup structure comprises a base and a flexible cup arranged outside the base and connectable to the base. The flexible cup has a shape conforming to that of the base and encompasses the base and a periphery of the user's buttocks. The defecating device cup structure further comprises a cup space adjusting unit, the cup space adjusting unit being able to adjust a space between the flexible cup and the user. By providing the cup space adjusting unit in the flexible cup, a portion of the flexible cup in contact with the user may have a better tightness, whereby guaranteeing a good cleaning effect and achieving a better wear comfort.

## Description

### Field of the Invention

The present invention relates to the filed of sanitary ware, and in particular to a defecating device cup structure and a defecating device.

### Background of the Invention

With the development of economy and the improvement of people's living level, aging problems worldwide become increasingly prominent. Nowadays, a great number of senior citizens are not able to relieve themselves due to bedridden, inconvenience in action and incontinence. Living quality and spiritual life of these senior citizens are seriously influenced. Moreover, for those who need to stay in bed due to postoperative rehabilitation or special reasons, there is also a problem of cleaning defecation in time. Therefore, a design of a device capable of automatically and easily dealing with defecation or urination will become a key point of an emerging industry of elderly care and rehabilitation nursing.

Thereupon, the defecating device is developing. An existing defecating device mainly comprises of a body and a cup. The body includes an electric motor, a contaminant container, a water tank and other suitable components; the cup includes a base and a flexible material. The base is usually placed between both legs of a user. The base may be provided with a leakage slot, an inlet pipe, a discharge pipe and other suitable components. The flexible material may be in direct contact with buttocks of the user. In order to deal with the defecation or urination for a long period of time and also to facilitate actions such as roll-over on a bed for these groups of people, in a comparatively advanced product at present, the cup is designed as a wearable type. The buttocks of the user may be encompassed by the flexible material, in such a manner that the excrement from these groups could be sucked into a designated contaminant container, whereby dealing with defecation and urination for long hours so as to solve the problem that these groups are not able to relieve themselves. Thus, these groups could be kept clean without restraining physical activities thereof.

When cleaning the excrement in the base and the cup, the existing defecating device usually adopts manners of generating a negative pressure and rinsing with water. Moreover, in order to ensure the comfort of the wearer, the current wearable cup design is usually not attached tightly to the user's body. If the user's body moves, a gap may often occur between the cup and the user's body, such that the cup could not tightly encompass a portion that contacts the wearer's body. This may cause the following problems:
1) When the excrement in the cup is being sucked, an ideal negative pressure could not be generated inside a cavity of the cup, influencing a cleaning effect toward the contaminant.
2) When the wearer is defecating or being rinsed with fresh water, it is easy to cause lateral leakage of the generated contaminative fluid. The laterally leaked fluid may not only contaminate surroundings such as bed sheet, produce peculiar smell and spread bacteria, but also cause bedsore on skins of a patient who is in contact with the contaminants for long hours, seriously influencing the living quality of the patient.

However, if the cup tightly encompasses the portion that contacts the wearer's body, although it is possible to prevent the lateral leakage and also possible to facilitate the discharge to a certain degree, long-time wearing may bring a burden to the patient and cause uncomfortable feelings, seriously influencing the experience of the patient.

As could be seen, to solve the problem, it has become currently urgent to design a defecating device that is wearable with ease and has a good tightness when being rinsed.

### Summary of the Invention

Technical problems to be solved in the present invention resides in that, directing to above disadvantages in prior art, there is provided a defecating device cup structure and a defecating device. The defecating device cup structure may provide a good tightness between a flexible cup and a portion contacting a user's body by a cup space adjusting unit arranged on the flexible cup, whereby ensuring a better cleaning effect and achieving a better comfort for wearing.

In order to achieve said objectives, on one hand, the present invention provides a defecating device cup structure. The defecating device cup structure comprise a base and a flexible cup arranged outside the base and connectable to the base, the flexible cup having a shape conforming to that of the base and encompassing the base and a periphery of the user's buttocks. The defecating device cup structure further comprises a cup space adjusting unit, the cup space adjusting unit being able to adjust a space between the flexible cup and the user.

Preferably, the cup space adjusting unit comprises an air bag, the air bag being connected to the flexible cup, and the air bag is able to be inflated or deflated, such that flexible cup is expanded or released along with the air bag.

Preferably, the air bag is arranged inside or outside the flexible cup.

Preferably, the flexible cup is arranged to be a single overlapped piece and forms a cavity by self-overlapping, the cavity having openings for legs passing through and encompassing the base and the buttocks, the air bag having an integral structure formed of a single communication unit or having discrete structures formed of a plurality of different communication units, and the air bag being positioned at least in a lower peripheral area of the flexible cup.

Preferably, the air bag is made of medical grade polyvinyl chloride materials meeting the hygienic standards, or made of EVA materials meeting medical device standards.

Preferably, the base is formed as a double-layer structure with a hollow cavity, the cup space adjusting unit further comprises an inflation motor and an exhaust valve, the inflation motor being used to inflate the air bag, and the exhaust valve being used to deflate the air bag.

Preferably, the cup space adjusting unit further comprises a control module and a contaminant detection sensor, the control module being arranged to receive a detection signal from the contaminant detection sensor, when the control module determines that the contaminant detection sensor detects contaminants in the base based on the detection signal, the control module controlling the inflation motor to start inflating; and
the control module stores a predetermined inflating time, when the inflating time of the inflation motor reaches the predetermined inflating time, the control module controlling the inflation motor to stop inflating to keep the air pressure inside of the air bag constant.

Preferably, the contaminant detection sensor adopts an infrared sensor which is arranged in the hollow cavity of the base.

Preferably, the flexible cup is made of a flexible material having flexibility, waterproof performance and air permeability.

More preferably, the flexible cup has crumpled surfaces.

Preferably, an adjustable connection device is arranged on both sides of an area of the flexible cup corresponding to the user's waist, such that the flexible cup is adapted to the size of the user's waist.

On the other hand, the invention also provides a defecating device. The defecating device comprises a body structure and a cup structure, wherein, the cup structure adopts above-mentioned defecating device cup structure, and the body structure is connected to the base through the flexible cup.

Preferably, the defecating device further comprises a discharge unit, the discharge unit including a discharge motor and a discharge port communicating with the discharge pipe connector, the discharge motor being used to generate a negative pressure, such that the contaminants may be discharged out of the base and the flexible cup via the discharge port.

Preferably, the defecating device further comprises a rinsing unit, the rinsing unit including a water inlet port communicating with an inlet pipe connector, the water inlet port being used to inject fresh water to rinse the user and the flexible cup; after the cleaning is finished, the discharge motor generates the negative pressure, such that the contaminated water upon rinsing is discharged out of the base and the flexible cup via the discharge port.

Preferably, the defecating device further comprises a drying unit, the drying unit including a heating motor and an air inlet arranged at a suitable position of the flexible cup and the base, the heating motor being used to blow the flexible cup with warm wind to dry the outside of the user, the base and the flexible cup.

The advantageous effects of the invention are as follows: by providing the cup space adjusting unit in the defecating device cup structure and connecting the inflatable air bag to the flexible cup, and with the cooperation of the inflation motor, the discharge unit, the rinsing unit and the drying unit, the improvement in tightness between the flexible cup and the user may be achieved without any wear burden to the user, whereby efficiently preventing lateral leakage, guaranteeing a good cleaning effect and achieving a better wear comfort, suitably for long time wearing for the patient.

### Brief Description of the Drawings

Figure 1 is a schematic perspective view showing a structure of a base according to a first embodiment of the present invention;
Figure 2 is a schematic view showing structures of a flexible cup and an air bag according to the first embodiment of the present invention; and
Figure 3 is a schematic view showing a structure of a body according to the first embodiment of the present invention.

### In reference numerals:

1 - flexible cup; 2 - nylon velcro; 3 - air bag; 10 - inlet pipe connector; 11 - discharge pipe connector; 12 - plate; 20 - control panel; 21 - water tank; 22 - pump; 23 - discharge motor; 24 - inlet pipe; 25 - contaminant container; 26 - discharge pipe.

### Detailed Description of the Embodiments

In order to better understand the inventive technical solutions for those skilled in the art, the defecating device cup structure and the defecating device according to the invention will be further described in detail with reference to accompanying drawings and specific embodiments.

### First Embodiment

There is provided a defecating device cup structure and a defecating device having the cup structure in this embodiment. The defecating device cup structure is capable of effectively addressing the lateral leakage problem due to the untight encompassment of the portion contacting the user's body (for example, the wear's body) by the flexible cup, whereby achieving a better cleaning effect and a better comfort for wearing. In the inventive defecating device cup structure, a portion of a flexible cup contacting a user's body may be provided with a sealed air bag, and a small eccentric inflation motor may automatically detect the contaminants to inflate or deflate the air bag. According to the present invention, the air bag may be in an inflated state or in a deflated state, whereby providing a tightness during the discharge of the contaminants and a comfort after the discharge of the contaminants.

In this embodiment, the defecating device cup structure may comprise a base and a flexible cup arranged outside the base and connectable to the base. The flexible cup may have a shape conforming to that of the base and encompass the base and a periphery of the user's buttocks. Herein, the defecating device cup structure may further comprise a cup space adjusting unit. The cup space adjusting unit can adjust a gap between the flexible cup and the user.

Herein, the flexible cup may be made of a flexible material having a thickness of 6-8mm. The flexible cup may be suitable for being worn and seated at a haunch, and also be able to follow the wearer's postures so as to facilitate the activities of the wearer on the bed. A cavity formed by the base may be used as an initial container for the excrement, and a cavity formed by the flexible cup may be used as a backup container for the excrement. In this embodiment, in order to achieve an ability to adjust the space between the flexible cup and the user, the flexible cup may be made of a flexible material having flexibility, waterproof performance and air permeability. Further, the flexible cup may have crumpled surfaces. Herein, the crumpled surfaces mean that the flexible cup is arranged to have wavy or folded structure so as to be stretchable to achieve a space adjustable effect.

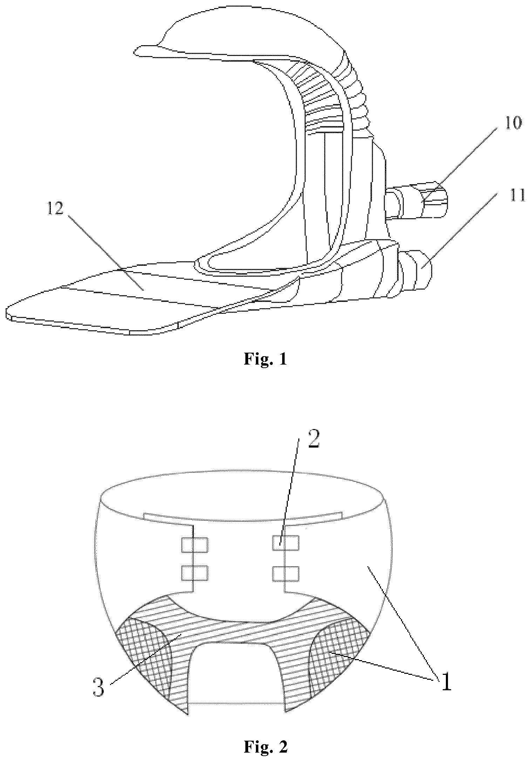

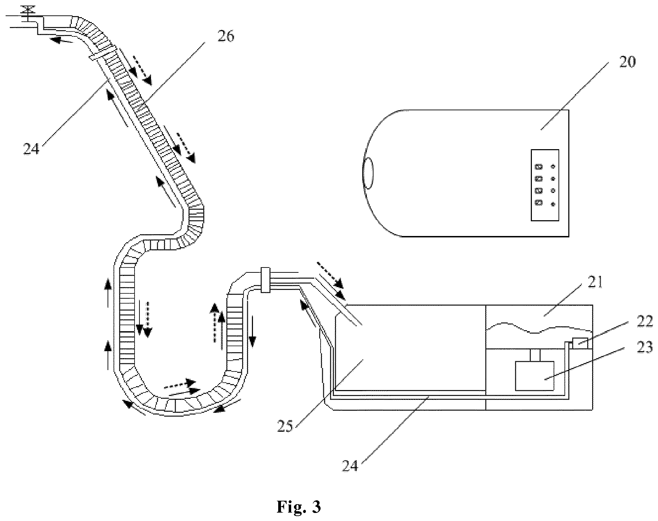

As shown in Figure 1, the base structure may have the containing cavity which conforms to the curved haunch of the user so as to easily collect the excrement, and may reserve a discharge pipe connector 11 for discharging contaminants and an inlet pipe connector 10 for cleaning. The base is usually seated between both legs of the user, such that the plate 12 may be positioned in an area of the user's buttocks. Since the plate 12 is positioned in an area of the user's buttocks, on one hand the base may be easily seated in place and on the other hand the contaminants may be easily discharged through a discharge pipe 26 (with reference to downward arrows in dashed lines in Figure 3).

In particular, the cup space adjusting unit may include the air bag which is connected to the flexible cup. The air bag could be inflated or deflated, such that the flexible cup could be expanded or released along with the air bag. In this embodiment, in order not to bring uncomfortable feelings to the wearer in use due to the tight attachment to the skins, the air bag may be arranged outside the flexible cup, that is, the air bag may be arranged on an outer surface of the flexible cup. When the air bag is inflated, it may fill the space between the flexible cup and the base, so as to achieve a tightly sealed effect between the flexible cup and the outside of the wearer. In Figure 2 showing the flexible cup, blank areas refer to outside of the flexible cup 1, checked areas refer to inside of the flexible cup 1, and diagonal areas refer to the air bag 3 outside of the flexible cup 1.

Dependent on wearers' shapes, flexible cups 1 may have different sizes. The overall length of the flexible cup 1 may be predetermined. Meanwhile, an adjustable belt may be provided in an area of the flexible cup 1 corresponding to the wearer's waist, in order to easily adjust the encompassed space. For example, an adjustable connection device may be provided on both sides of the area of the flexible cup 1 corresponding to the user's waist, such that the flexible cup 1 may be adapted to the size of the user's waist to assist in adjusting the space between the flexible cup 1 and the user to a certain degree. In the invention, the adjustable connection device may be a nylon Velcro 2. As a matter of course, it should be readily appreciated by those skilled in the art that, the nylon Velcro 2 may be replaced with any other suitable devices as long as the flexible cup 1 can be formed as a cavity and the perimeter of the cavity corresponding to the user's waist is adjustable.

In order to be easily worn by the bedridden patient, the flexible cup 1 may be arranged to be a single overlapped piece and form a cavity by self-overlapping. The cavity may have openings for legs passing through and may encompass the base and the buttocks (similarly to a diaper for infants commonly used in daily life). The air bag 3 may have an integral structure formed of a single communication unit or may have discrete structures formed of a plurality of different communication units. The air bag 3 is positioned at least in a lower peripheral area of the flexible cup 1. Herein, the lower peripheral area of the flexible cup 1 refers to an area in which, when the flexible cup 1 is overlapped by the nylon Velcro 2 and the base together with the wear's buttocks and legs are encompassed within the space formed thereby, the flexible cup 1 contacts the user's legs. It also means that the air bag 3 may be provided in any other peripheral areas of the overall flexible cup 1 except for the waist area. In addition, the air bag 3 on the flexible cup 1 may be disposed along the lower peripheral area of the flexible cup 1 to form a single communicated integral structure (which could be inflated by an inflation motor). The air bag 3 may also be formed of a plurality of non-communicated discrete structures, which may be disposed in the lower peripheral area of the flexible cup 1 or even in an area of the flexible cup 1 corresponding to the user's buttocks (non-communicated discrete portions of the air bag 3 are necessarily inflated by separate inflation motors; although this is relatively expensive, the reliability may be improved). Under normal circumstances, when the cup structure is worn, the lateral leakage may highly possibly occur in the leg area. The problem of the lateral leakage may be specially solved by providing the air bag 3 along the lower peripheral area of the flexible cup 1 contacting the user.

Since a volume of the air bag 3 is expandable with the amount of the air therein (in particular, the air bag 3 has a larger volume in the inflated state than in the deflated state), materials of the air bag 3 are required to have a certain expandability. In addition, the air bag 3 may further adopt a form of creased structure to guarantee the deformability of its volume. Meanwhile, since the contaminants are normally rinsed with water, the materials of the air bag 3 may have waterproof performance to a certain degree. Preferably, in order to guarantee the inflated effect of the air bag 3 while ensuring the security and the comfort provided by the portions of the air bag 3 contacting the wearer, the air bag 3 may be made of medical grade polyvinyl chloride materials meeting the hygienic standards, or made of EVA materials meeting medical device standards.

In order to increase a rate of inflation and deflation, the volume of the air bag 3 could not be excessively large, which preferably ensures the inflation or the deflation can be completed within 30 seconds. As a matter of course, the volume of the air bag 3 may be predetermined dependently on groups or users' shapes and shall not be restrained herein.

Under normal circumstances, with reference to Figure 1, a bottom of the base (corresponding to the area between the discharge pipe connector 11 and the plate 12) may be formed as a double-layer structure with a hollow cavity. The cup space adjusting unit may further comprise an inflation motor and an exhaust valve (not shown in Figure 1). The inflation motor is arranged in the hollow cavity of the base. The inflation motor may be used to inflate the air bag. The exhaust valve may be used to deflate the air bag. In order to guarantee the rate of inflation and a short connecting line of the inflation motor, the inflation motor may preferably be a small eccentric motor so as to be easily arranged in the hollow cavity of the base. The small eccentric motor usually has a supply voltage of 5V which is safe for the user and can not cause noise pollution.

The cup space adjusting unit may further comprise a control module and a contaminant detection sensor. The control module may be arranged to receive a detection signal from the contaminant detection sensor. When the control module determines that the contaminant detection sensor detects contaminants in the base based on the detection signal, the control module may control the inflation motor to start inflating. Also, the control module may store a predetermined inflating time. When the inflating time of the inflation motor reaches the predetermined inflating time, the control module may control the inflation motor to stop inflating. The cup space adjusting unit may further comprise a pressure sensor. After the pressure inside of the air bag reaches a predetermined pressure, the control module may control the inflation motor to stop inflating. In this manner, the air pressure inside of the air bag may be kept constant. With reference to Figure 3, in order to further simplify the structure, the control module may be built in the control panel 20 of the defecating device.

Herein, the contaminant detection sensor may adopt an infrared sensor which is arranged in the hollow cavity of the base. In the case of the infrared sensor, a temperature change inside the base may be detected to determine the presence of excrement (for example, urination and defecation) instantly. The excrement usually has a certain temperature when getting out, which could be detected by the infrared sensor. As a matter of course, any other sensors besides the infrared sensor may be used to detect the presence of the contaminants and shall not be restrained herein.

In this embodiment, the infrared sensor may perceive the temperature inside the base, so as to detect the presence of the excrement then to activate and start the discharging operation. Prior to the start of discharging, the inflation motor may first inflate the air bag to retain the air pressure therein. After the completion of the discharging operation, the air bag may be deflated to restore to the released state. Therefore, the pressure applied to the contacted skins of the patient could be reduced to relieve the burden to the wearer.

In this embodiment, since the inflatable air bag is attached outside the flexible cup formed of the flexible materials, the air bag may fill the space between the base, the flexible cup and the skins of patient after being inflated by the inflation motor, advantageously preventing the leakage of contaminants and contaminative fluid. Meanwhile, the tightness of the cavity formed by the flexible cup may be improved and the negative pressure inside of the flexible cup may be increased.

Under normal circumstances, the automatic defecating device may have a control module for controlling the discharging operation and a discharge motor. Therefore, there are two ways to inflate the air bag 3. In one way, it is inflated by the small eccentric motor (i.e., aforesaid inflation motor), which has relatively high efficiency and security. In the other way, it is inflated by the discharge motor via an air inlet. In this case, the cost may be lowered due to omission of the separate inflation motor. The deflation of the air bag 3 may be controlled by an exhaust valve (for example, an electronic control valve). Other structures of the defecating device in relation to the discharging operation will be described in detail below.

In particular, the automatic defecating device may comprise a body structure and aforesaid cup structure. The body structure may be connected to the base through the flexible cup. In order to achieve an automatic clearance of the contaminants, the body structure of the defecating device may include a discharge unit. The discharge unit may include a discharge motor 23 and a discharge port (not shown in Figure 2) communicating with the discharge pipe connector 11. The discharge motor 23 is used to generate a negative pressure, such that the contaminants may be discharged out of the flexible cup via the discharge port. Herein, discharge port may be a pipe connecting the flexible cup to a contaminant container 25, so as to convey the contaminants from the cavity formed by the flexible cup to the contaminant container 25. The discharge motor may be used to generate a negative pressure in the cavity of the flexible cup, such that the contaminants may be sucked to the contaminant container 25 on the lower side of the base via the discharge port to be discharged out of the base and the flexible cup.

After the contaminants are discharged, it is possible to further rinse the user and the flexible cup to keep clean and hygienic. Accordingly, the body structure of the defecating device may further include a rinsing unit. The rinsing unit may comprise a water tank 21 (completely isolated from the contaminant container 25) and a water inlet port (not shown in Figure 2) communicating with an inlet pipe connector 10. The water inlet port may be used to inject fresh water to rinse the defecating portion of the user and the cavity of flexible cup following completion of the discharging operation. In particular, the fresh water stored in the water tank 21 may be guided to the base via an inlet pipe 24 (connected to the inlet pipe connector 10) by a pump 22 (with reference to upward arrows in solid lines in Figure 3), so as to rinse the user and the flexible cup. The discharge motor 23 may generate a negative pressure, such that the contaminated water upon rinsing may be discharged out of the base and the flexible cup into the contaminant container 25 through the discharge pipe 26 connected to the discharge pipe connector 11 (with reference to downward arrows in solid lines in Figure 3) and via the discharge port. It should be understood that, one end of the inlet pipe 24 may be connected to the inlet pipe connector 10 and the other end thereof may be connected to any other clean water supply (for example, commercially available water faucet) besides the water tank 21. In this case, the water tank 21 and the pump 22 may be omitted.

After the user and the flexible cup are rinsed, in order to keep the user and the flexible cup waterless, the body structure of the defecating device may further comprise a drying unit. The drying unit may include a heating motor and an air inlet (not shown in Figure 2) arranged at a suitable position of the flexible cup and the base. The heating motor may be used to blow the flexible cup with warm wind to dry the user and the flexible cup. In particular, the heating motor may convey the warm wind into the cavity formed of the flexible cup via the air inlet to dry the rinsed portion of the user, the base and the flexible cup.

Herein, a duration time of the negative pressure generated by the discharge motor in the discharging process and a working time of the pump in the rinsing process may be controlled by presetting time values in the control module.

In this embodiment, since the cavity formed of the flexible cup may have different volumes depending on the inflated state and the deflated state of the air bag, in order not to influence normal use of the bedridden patient, a venting port of the air bag is preferably positioned closely to the air inlet and the discharge port, for example, in the vicinity of a portion of the flexible cup connected to the discharge pipe.

The use of the defecating device will be described below. The base is first seated at a suitable position for the user, and then the flexible cup is worn such that the flexible cup is able to encompass the base and the user's buttocks; then the nylon Velcro of the flexible cup is adjusted so that the entire defecating device cup structure can be kept in place. For example, the encompassment extent of the flexible cup to the user's waist can be adjusted by bonded positions of the nylon Velcro. The flexible cup may be in loose contact with the skins of the user's haunch and buttocks (it may be looser than ordinary underwear), so that less wear burden will be brought to the patient. When the infrared sensor detects that the user does not relieve himself or herself, the flexible cup may be kept in current state; when the infrared sensor detects that the user relieves himself or herself, the defecating device cup structure may activate a discharge process in the following sequence.

First, the control module receives the detection signal and activates the inflation motor to inflate the air bag. Since the overall length of the lower peripheral area of the flexible cup is fixed, the inflated air bag may fill the space between the lower peripheral area of the flexible cup and the user, whereby achieving a tight effect.

When a predetermined inflating time is reached, the inflation motor may stop inflating and automatically close the valve to retain the air pressure inside the air bag.

In the half way of the inflation process, the discharge motor starts to work to generate the negative pressure in the cavity formed of the flexible cup, so as to suck the user's excrement into the designated contaminant container.

After the excrement is cleaned, the fresh water is injected into the cavity formed of the flexible cup via the inlet pipe communicating with the water inlet port of the flexible cup to rinse the user's buttocks and the flexible cup, and then the discharge motor is activated again to generate the negative pressure in the cavity formed of the flexible cup, so as to discharge the contaminated water upon rinsing.

After the contaminated water is discharged, the valve of the air bag (i.e., exhaust valve) is opened to deflate the air bag, such that the flexible cup is restored to the initial loose state.

Then the heating motor is activated to dry the rinsed the buttocks and the flexible cup. The whole discharge process is finished.

It should be understood, in this embodiment, the reason why the defecating device activates the discharge motor in the half way of the inflation process resides in that, the air bag is in a complete expansion state after being inflated, such that an enclosed space could be formed between the flexible cup and the user; if the discharge motor is activated at this point, a great negative pressure would instantaneously be generated in the enclosed space, which may bring a serious uncomfortable feeling to the patient and which also may impact on the service life of the discharge motor. By contrast, in the manner where the discharge motor is activated in the half way of the inflation process, not only a better discharge effect may be achieved and the comfort of the user may be improved, but also the service life of the discharge motor may be prolonged.

In the defecating device cup structure according to this embodiment, by arranging the volume adjustable air bag in the flexible cup and inflating and deflating the air bag by the inflation motor, the tightness between the flexible cup and the user may be efficiently guaranteed. Therefore, the defecating device cup structure could efficiently be prevented from the fluid lateral leakage in the process of cleaning the excrement, while an improved comfort may be provided for the user.

### Second Embodiment

In this embodiment, there is provided a defecating device cup structure and a defecating device having the cup structure. The difference between the defecating device cup structure of this embodiment and that of the first embodiment resides in the position of the air bag.

In the defecating device cup structure according to this embodiment, the air bag in a cup space adjusting unit is arranged inside the flexible cup. Compared with the case that the air bag is arranged outside the flexible cup according to the first embodiment, after the air bag of this embodiment is inflated, the flexible cup may more directly exert an adjustablity effect on the inside space and have a better tightness, but at this point, the air bag may directly apply a stress to the user, whereby providing a decreased comfort.

The structure of the air bag in the defecating device cup structure according to this embodiment and structures of other components are the same as those in the first embodiment, and thus detailed description thereof will be omitted herein.

In the defecating device cup structure according to the first and the second embodiments, in order to solve the problem of lateral leakage, improvements have been made to the existing structure of the flexible cup. According to the inventive embodiments, by providing the cup space adjusting unit in the defecating device cup structure and connecting the inflatable air bag to the flexible cup, and with the cooperation of the inflation motor, the discharge unit, the rinsing unit and the drying unit, the improvement in tightness between the flexible cup and the user may be achieved without any wear burden to the user, whereby efficiently preventing lateral leakage, guaranteeing a good cleaning effect and achieving a better wear comfort, suitably for a long time wearing for the patient.

It should be understood that the above embodiments are merely exemplary embodiments for the purpose of illustrating the principle of the invention, and the invention is not limited thereto. Various modifications and improvements can be made by a person having ordinary skill in the art without departing from the spirit and the essence of the invention. Accordingly, all of the modifications and improvements will also fall into the protection scope of the invention.

## Claims

1. A defecating device cup structure, comprising:
a base; and
a flexible cup arranged outside the base and connectable to the base, the flexible cup having a shape conforming to that of the base and encompassing the base and a periphery of the user's buttocks,
wherein, the defecating device cup structure further comprises a cup space adjusting unit, the cup space adjusting unit being able to adjust a space between the flexible cup and the user.

2. The defecating device cup structure according to claim 1, wherein,
the cup space adjusting unit comprises an air bag, the air bag being connected to the flexible cup, and the air bag is able to be inflated or deflated, such that flexible cup is expanded or released along with the air bag.

3. The defecating device cup structure according to claim 2, wherein,
the air bag is arranged inside or outside the flexible cup.

4. The defecating device cup structure according to claim 3, wherein,
the flexible cup is arranged to be a single overlapped piece and forms a cavity by self-overlapping, the cavity having openings for legs passing through and encompassing the base and the buttocks, the air bag having an integral structure formed of a single communication unit or having discrete structures formed of a plurality of different communication units, and the air bag being positioned at least in a lower peripheral area of the flexible cup.

5. The defecating device cup structure according to claim 2, wherein,
the air bag is made of medical grade polyvinyl chloride materials meeting the hygienic standards, or made of EVA materials meeting medical device standards.

6. The defecating device cup structure according to claim 2, wherein,
a bottom of the base is formed as a double-layer structure with a hollow cavity, the cup space adjusting unit further comprises an inflation motor and an exhaust valve, the inflation motor being used to inflate the air bag, and the exhaust valve being used to deflate the air bag.

7. The defecating device cup structure according to claim 6, wherein,
the cup space adjusting unit further comprises a control module and a contaminant detection sensor, the control module being arranged to receive a detection signal from the contaminant detection sensor, when the control module determines that the contaminant detection sensor detects contaminants in the base based on the detection signal, the control module controlling the inflation motor to start inflating; and
the control module stores a predetermined inflating time, when the inflating time of the inflation motor reaches the predetermined inflating time, the control module controlling the inflation motor to stop inflating to keep the air pressure inside of the air bag constant.

8. The defecating device cup structure according to claim 7, wherein,
the contaminant detection sensor adopts an infrared sensor which is arranged in the hollow cavity of the base.

9. The defecating device cup structure according to any one of claims 1-8, wherein,
the flexible cup is made of a flexible material having flexibility, waterproof performance and air permeability.

10. The defecating device cup structure according to any one of claims 1-8, wherein,
the flexible cup has crumpled surfaces.

11. The defecating device cup structure according to any one of claims 1-8, wherein,
an adjustable connection device is arranged on both sides of an area of the flexible cup corresponding to the user's waist, such that the flexible cup is adapted to the size of the user's waist.

12. A defecating device, comprising a body structure and a cup structure, wherein,
the cup structure adopts the defecating device cup structure according to any one of claims 1-11, in which the base and the body structure is connected to the base through the flexible cup.

13. The defecating device according to claim 12, wherein,
the defecating device further comprises a discharge unit, the discharge unit including a discharge motor and a discharge port communicating with the discharge pipe connector, the discharge motor being used to generate a negative pressure, such that the contaminants may be discharged out of the base and the flexible cup via the discharge port.

14. The defecating device according to claim 13, wherein,
the defecating device further comprises a rinsing unit, the rinsing unit including a water inlet port communicating with an inlet pipe connector, the water inlet port being used to inject fresh water to rinse the user and the flexible cup; after the cleaning is finished, the discharge motor generates the negative pressure, such that the contaminated water upon rinsing is discharged out of the base and the flexible cup via the discharge port.

15. The defecating device according to claim 14, wherein,
the defecating device further comprises a drying unit, the drying unit including a heating motor and an air inlet arranged at a suitable position of the flexible cup and the base, the heating motor being used to blow the flexible cup with warm wind to dry the outside of the user, the base and the flexible cup.
